# DEMANDE DE BREVET EUROPEEN

(11) **EP 4 840 396 A1**
(43) Date de publication de la demande: **18.11.2026**
(21) Numéro de dépôt: 26177844.3
(22) Date de dépôt: 11.05.2026
(51) Int. Cl.: A61B 5/145

(54) **DISPOSITIF DE DÉTECTION D'UN RAYONNEMENT RÉTRODIFFUSÉ PAR UN OBJET**

(30) Priorité: 12.05.2025 FR 2505008
(71) Demandeur: Commissariat à l'Energie Atomique et aux Energies Alternatives, 75015 Paris (FR)
(72) Inventeur: ANDRE, LUC, 38054 Grenoble cedex 09 (FR); BLANQUER, GUILLAUME, 38054 Grenoble cedex 09 (FR); LEJOSNE, MARTIN, 38054 Grenoble cedex 09 (FR)
(74) Mandataire: INNOV-GROUP

(57) **Abrégé**

Dispositif de mesure d'un rayonnement rétrodiffusé (12, 12₁, 12₂, 12₃, 12₄) par un objet (2) comportant :
- au moins une source de lumière (10) configurée pour émettre un faisceau lumineux (11), , vers une surface (S) de l'objet, formant une zone d'illumination ;
- un photodétecteur (22, 22₁, 22₂, 22₃, 22₄), pour détecter le rayonnement rétrodiffusé par l'objet sur une surface de détection;
le dispositif comportant :
- un collecteur de rayonnement (20, 20₁, 20₂, 20₃, 20₄), s'étendant selon une surface de collection décrivant au moins un secteur annulaire, et plus grande que la surface de détection ;
- la surface de collection étant disposée face ou contre la surface de l'objet,
- de façon que le collecteur est agencé pour renvoyer le rayonnement rétrodiffusé (12, 12₁, 12₂, 12₃, 12₄), incident à la surface de collection, vers le photodétecteur.

## Description

### DOMAINE TECHNIQUE

Le domaine technique de l'invention est la mesure d'un rayonnement rétrodiffusé par un objet, par exemple un tissu corporel vivant.

### ART ANTERIEUR

La spectrométrie de réflectance diffuse, souvent désignée par l'acronyme anglais DRS (Diffuse Reflectance Spectroscopy), consiste à exploiter la lumière rétrodiffusée par un objet diffusant soumis à un éclairement, généralement ponctuel. Cette technique s'avère performante pour caractériser des propriétés optiques d'objets, en particulier les propriétés de diffusion ou d'absorption.

Mise en œuvre sur la peau, cette technique permet par exemple de caractériser la peau, comme décrit dans le document EP2762064. Les auteurs de ce document décrivent une sonde de mesure destinée à être appliquée contre la peau. Cette sonde comporte une fibre optique centrale, dite fibre d'excitation, destinée à diriger un faisceau lumineux vers un objet de peau. Des fibres optiques, disposées autour de la fibre centrale, dites fibres de détection, recueillent un signal optique rétrodiffusé par la peau. Des moyens d'analyse spectrale du signal optique ainsi collecté, couplés à des algorithmes de calcul, permettent d'estimer des paramètres du derme, en particulier la concentration de certains chromophores, par exemple l'oxyhémoglobine ou la désoxyhémoglobine, mais aussi des paramètres gouvernant le parcours des photons dans le derme, notamment le coefficient de diffusion réduit µs' ainsi que le coefficient d'absorption µₐ. Les applications sont nombreuses, et peuvent par exemple permettre de détecter et de quantifier des concentrations d'éléments dans la peau, ou d'accéder à une information relative à l'oxymétrie des vaisseaux sanguins traversant la peau, ou encore des paramètres respiratoires, telles que la fréquence cardiaque. Des applications sont décrites dans EP2762064, ainsi que dans la publication Haahr R.,"An Electronic Patch for Wearable Health Monitoring by Reflectance Pulse Oxymetry", IEEE Transactions on biomedical circuits and systems, 2011, ou encore dans les brevets EP4394360, EP3311138, EP3236241, EP3210531, EP3338632, ou encore WO2022076381.

Selon les principes de la diffusion de la lumière dans un milieu diffusant, la profondeur moyenne de pénétration des photons dans un objet diffusant augmente avec l'écartement entre la fibre d'excitation et la fibre de détection. Autrement dit, plus la distance entre la fibre d'excitation et la fibre de détection augmente, plus le résultat est représentatif des propriétés optiques des couches profondes du milieu examiné. A l'inverse, plus cette distance diminue, plus le résultat est représentatif des couches superficielles du milieu diffusant.

US6031603 décrit un dispositif fibré de type oxymétrie de pouls, les fibres étant destinées à venir au contact d'une zone corporelle.

US20180017772 décrit un dispositif d'analyse d'un faisceau rétrodiffusé par un objet, avec un détecteur placé selon une configuration confocale. Le faisceau rétrodiffusé par l'objet est renvoyé vers un photodétecteur, sous la forme d'un faisceau collimaté, par une portion annulaire d'une lentille.

Le document EP3054281 décrit un dispositif comportant un système optique entre des fibres optiques et un objet à caractériser, par exemple la peau d'un individu. Selon ce dispositif, les fibres optiques sont disposées à distance de l'objet à caractériser. Un système optique, de grandissement supérieur à 1, est disposé entre les fibres et l'objet à caractériser. Le système optique, disposé à distance de l'objet caractérisé, assure un couplage optique des fibres optiques avec des zones élémentaires, ponctuelles, à la surface de l'objet.

Un dispositif plus compact a été décrit dans EP350266. Ce dispositif comporte un capteur d'image relié à un faisceau de fibres optiques, ce dernier étant appliqué directement contre l'objet. Le faisceau de fibres optiques est configuré pour transporter une image du rayonnement rétrodiffusé émanant de la surface de l'objet vers la matrice de pixels du capteur d'image. Cependant, selon une telle configuration, la distance, parallèlement à la surface de l'objet, entre le centre de la source de lumière et la matrice de pixels ne peut pas être inférieure à 3 ou 4 mm.

Les inventeurs proposent un dispositif plus sensible que ceux décrits dans l'art antérieur. Il s'agit notamment d'améliorer le rapport signal sur bruit de l'intensité du rayonnement rétrodiffusé détecté. La résolution du dispositif, c'est-à-dire la capacité à détecter des variations fines de composition de l'objet analysé, varie comme la racine carrée de l'intensité du rayonnement rétrodiffusé détecté. Il est donc important d'augmenter l'intensité du rayonnement rétrodiffusé détecté, tout en minimisant le bruit de détection.

Le dispositif proposé est également bien adapté à une source de lumière cohérente, en présentant une moindre sensibilité à la formation de figures de diffraction à la surface de l'échantillon, du fait d'interférences constructives et destructives.

### EXPOSE DE L'INVENTION

Un premier objet de l'invention est un dispositif de mesure d'un rayonnement rétrodiffusé par un objet comportant :
- au moins une source de lumière configurée pour émettre un faisceau lumineux, dans une bande spectrale d'illumination, vers une surface délimitant l'objet, de façon à former, sur ladite surface, une zone d'illumination;
- un photodétecteur, pour détecter le rayonnement rétrodiffusé par l'objet, lorsque ce dernier est illuminé par la source de lumière, le photodétecteur s'étendant selon une surface de détection ;
le dispositif étant caractérisé en ce qu'il comporte :
- un collecteur de rayonnement, s'étendant selon une surface de collection, la surface de collection décrivant au moins un secteur annulaire s'étendant autour d'un centre, la surface de collection étant plus grande que la surface de détection ;
- et en ce que lorsque la surface de collection est disposée face ou contre la surface de l'objet, le collecteur de rayonnement est configuré pour renvoyer un rayonnement, incident à la surface de collection, vers le photodétecteur, le collecteur étant optiquement couplé au photodétecteur ;
- de façon que lorsque le centre, autour duquel s'étend le collecteur, est disposé dans la zone d'illumination, le collecteur est agencé pour renvoyer le rayonnement rétrodiffusé, incident à la surface de collection, vers le photodétecteur.

Selon une possibilité, le ou chaque collecteur est formé d'une plaque de zone de Fresnel, le collecteur étant formé de portions concentriques alternant entre des portions transparentes et opaques, configurées pour focaliser le rayonnement incident au collecteur vers le photodétecteur auquel ledit collecteur est optiquement couplé.

Selon une possibilité, le ou chaque collecteur est formé de portions concentriques alternant entre des portions respectivement d'un premier indice et d'un deuxième indice, supérieur au premier indice, et configurées pour focaliser le rayonnement incident au collecteur vers le photodétecteur auquel ledit collecteur est optiquement couplé.

Selon une possibilité, le ou chaque collecteur est formé est formé d'une structure optique, de type métasurface ou cristaux photoniques bidimensionnels, configurée pour focaliser le rayonnement incident au collecteur vers le photodétecteur auquel ledit collecteur est optiquement couplé.

Le collecteur peut s'étendre à une distance, dite distance de rétrodiffusion, du centre C, de façon à renvoyer le rayonnement rétrodiffusé par l'objet, à ladite distance de rétrodiffusion, vers le photodétecteur.

Selon une possibilité, le dispositif comporte :
- plusieurs photodétecteurs ;
- plusieurs collecteurs concentriques, s'étendant autour du centre, chaque collecteur étant disposée à une distance du centre différente des autres collecteurs, chaque collecteur étant optiquement couplé à un photodétecteur différent.

Chaque collecteur est ainsi configuré pour collecter un rayonnement rétrodiffusé par l'objet à une distance de rétrodiffusion qui correspond à la distance entre le collecteur et le centre, c'est-à-dire au rayon du collecteur.

La surface de collection peut être inférieure à 10 mm² ou à 5 mm² ou à 1 mm².

Chaque photodétecteur peut être disposé face au collecteur auquel il est couplé.

La largeur du ou de chaque collecteur, radialement, est inférieure à 500 µm ou 250 µm ou 100 µm. Par radialement, on entend selon un rayon du collecteur passant par le centre.

Selon une possibilité:
- au moins un collecteur est subdivisé en plusieurs collecteurs élémentaires, s'étendant à une même distance du centre ;
- chaque collecteur élémentaire est optiquement couplé à un photodétecteur élémentaire.
Ainsi, chaque photodétecteur élémentaire forme le photodétecteur.

Chaque collecteur élémentaire peut s'étendre selon une extension angulaire, autour du centre, inférieure à 90° ou inférieure à 45° ou inférieure à 30° ou inférieure à 15°.

Selon une possibilité, le dispositif comporte un déflecteur, configuré pour dévier, le faisceau lumineux, atteignant l'objet, vers au moins un collecteur.

La bande spectrale d'illumination peut s'étendre dans le domaine visible ou dans l'infra-rouge. Un deuxième objet de l'invention est un procédé de détection d'un rayonnement rétrodiffusé par un objet, à l'aide d'un dispositif selon le premier objet de l'invention, le procédé comportant les étapes suivantes :
- a) placement du dispositif sur l'objet, de façon que le centre soit situé dans la zone d'illumination et que le ou chaque collecteur soit disposé contre ou face à la surface de l'objet ;
- b) illumination de l'objet à l'aide de la source de lumière, de façon à former, sur la surface de l'objet, une zone d'illumination,
- c) détection, par le ou chaque photodétecteur, du rayonnement rétrodiffusé par l'objet renvoyé par le collecteur optiquement couplé audit photodétecteur.

Le procédé peut comporter une détermination d'une propriété optique de diffusion et/ou d'absorption en fonction d'une intensité du rayonnement rétrodiffusé, à différentes distances de rétrodiffusion.

Le procédé peut comporter une détermination d'une concentration d'une espèce absorbant la lumière dans la bande spectrale d'illumination.

L'invention sera mieux comprise à la lecture de l'exposé des exemples de réalisation présentés, dans la suite de la description, en lien avec les figures listées ci-dessous.

### FIGURES

La figure 1A montre un exemple de dispositif dans un plan XZ perpendiculaire à la surface de l'objet.
La figure 1B montre l'exemple de dispositif dans un plan XY parallèle à la surface de l'objet.
La figure 1C est une vue d'un exemple de dispositif dans un plan XZ perpendiculaire à la surface de l'objet.
La figure 1D est une vue d'un exemple de dispositif dans un plan XZ perpendiculaire à la surface de l'objet.
La figure 2A montre une variante du dispositif de la figure 1A, comportant un déflecteur.
La figure 2B schématise le déflecteur représenté sur la figure 2A.
La figure 3A est une modélisation de parcours de photons dans un objet.
La figure 3B montre l'angle d'un rayonnement rétrodiffusé par rapport à la direction normale à la surface de l'objet pour deux types de sources de lumière (collimatée ou lambertienne), avec et sans déflecteur.
La figure 4 montre un exemple de collecteur de type plaque de zone de Fresnel.
La figure 5A représente l'obtention d'un collecteur tel que représenté sur la figure 4.
La figure 5B montre la position d'un photodétecteur par rapport à une plaque de zone de Fresnel
La figure 5C montre l'intensité lumineuse focalisée sur un photodétecteur par une plaque de zone de Fresnel.
La figure 5D est un profil d'intensité le long d'une ligne de la figure 5C passant par le centre de l'image.
La figure 6 montre les longueurs des trajectoires moyennes parcourues par les photons dans l'objet représenté sur la figure 1A.
La figure 7 montre une variante, selon laquelle chaque collecteur est subdivisé en collecteurs élémentaires situés à une même distance d'un même centre, chaque collecteur élémentaire étant optiquement couplé à un même détecteur élémentaire.
La figure 8 montre un circuit de traitement configuré pour recevoir des signaux de détection générés par deux photodétecteurs.

### EXPOSE DE MODES DE REALISATION PARTICULIERS

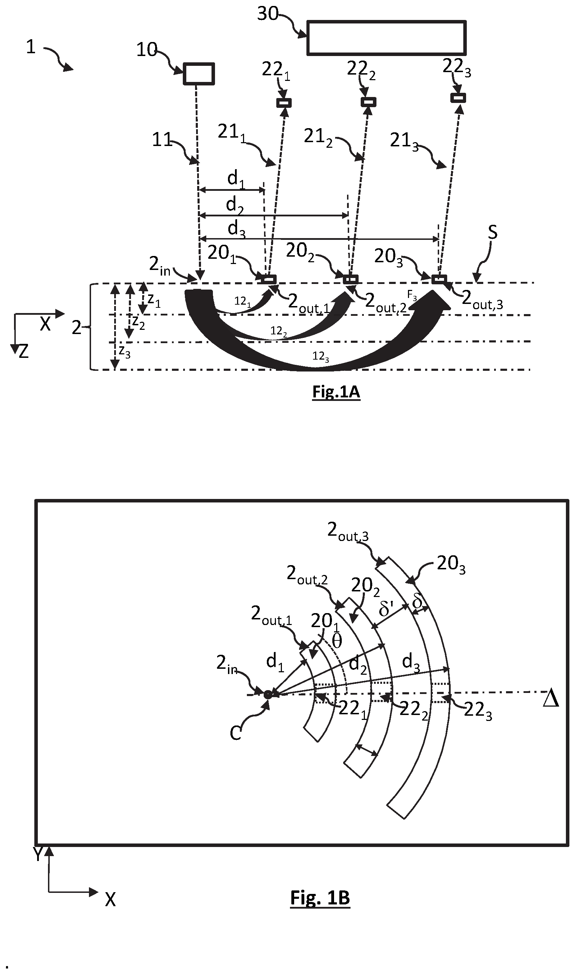

Les figures 1A à 1D schématisent les principaux composants d'un dispositif 1 selon l'invention. Le dispositif 1 comporte une source de lumière 10, configurée pour émettre un faisceau d'illumination 11 vers la surface S d'un objet 2. Le faisceau d'illumination est émis dans une bande spectrale d'illumination Δλ. De préférence, la bande spectrale d'illumination est étroite, et s'étend sur moins de 10 nm ou moins de 5 nm. La source de lumière 10 peut être une source laser ou une LED couplée à un filtre passe bande définissant la bande spectrale d'illumination. La bande spectrale d'illumination peut s'étendre dans le domaine visible ou dans le domaine infra-rouge.

Par exemple, lorsqu'on cherche à déterminer une concentration de glucose dans l'objet, la bande spectrale d'illumination est dans le proche infra-rouge. La section efficace d'absorption des tissus est généralement faible, et le glucose présente des bandes spectrales d'absorption, par exemple à 1540 nm.

Le faisceau d'illumination 11 forme, à la surface S de l'objet, une trace lumineuse, dite zone d'illumination 2ᵢₙ. La zone d'illumination 2ᵢₙ correspond à une intersection entre le faisceau d'illumination 11 et la surface S de l'objet. Dans l'exemple représenté, la zone d'illumination est considérée comme ponctuelle, ou considérée comme telle, ce qui est le mode de réalisation préféré. Elle s'étend de préférence selon un diamètre ou une plus grande diagonale inférieure à 1 mm.

L'objet 2 peut être une zone corporelle d'un être vivant, par exemple de la peau, de façon à caractériser les propriétés d'absorption ou de diffusion de la peau, ou d'estimer un paramètre physiologique, par exemple une teneur en oxygène, ou en glucose, ou une fréquence respiratoire, comme décrit dans les brevets cités dans l'art antérieur.

Sous l'effet de l'illumination, une partie de la lumière provenant du faisceau d'illumination 11 diffuse dans l'objet 2, et est rétrodiffusée par ce dernier de façon à former un rayonnement rétrodiffusé émanant de la surface S de l'objet. Comme décrit dans l'art antérieur, le rayonnement rétrodiffusé peut être détecté, ce qui permet de déterminer des propriétés optiques de l'objet, par exemple des propriétés optiques de diffusion ou d'absorption. On peut également ou alternativement estimer la concentration d'une espèce cible, absorbant la lumière, dans la bande spectrale d'illumination, dans l'objet. Une telle espèce cible est désignée par la suite chromophore. Il peut s'agir d'une molécule, par exemple du glucose, ou de l'oxygène, ou du CO₂, dont on souhaite déterminer la concentration dans l'objet analysé.

Par propriété optique de diffusion ou d'absorption, il est entendu un coefficient d'absorption, ou un coefficient de diffusion, ou un coefficient de diffusion réduit, ces grandeurs étant connues de l'homme du métier, et décrites dans la demande EP3054281.

Sur la figure 1A, on a schématisé, par des flèches, le parcours, dans l'objet 2, des photons diffusant dans ce dernier pour former le rayonnement rétrodiffusé. Le rayonnement rétrodiffusé émane de la surface S de l'objet 2 selon différentes zones de rétrodiffusion 2_{out,1} , 2_{out,2} , 2_{out, 3} distinctes de la zone d'illumination 2ᵢₙ. On désigne par le terme "distance de rétrodiffusion" une distance d séparant une zone de rétrodiffusion de la zone d'illumination 2ᵢₙ.

La figure 1A est une vue selon un plan XZ, ou X est un axe longitudinal et Z est un axe transversal, perpendiculaire à la surface S de l'objet 2. On a représenté trois zones de rétrodiffusion 2_{out,1} ,2_{out,2} ,2_{out, 3} s'étendant respectivement à trois distances de rétrodiffusion d₁, d₂ et d₃ de la zone d'illumination 2ᵢₙ, et desquelles émanent respectivement des rayonnements rétrodiffusés 12₁, 12₂, 12₃. Le terme zone de rétrodiffusion désigne une zone de la surface de l'objet dont la géométrie est telle que la zone peut être considérée comme étant située à une même distance de rétrodiffusion de la zone d'illumination 2ᵢₙ.

La figure 1B représente une vue du dispositif dans un plan XY, ou Y est un axe latéral. Le plan XY est parallèle à la surface S de l'objet. Chaque zone de rétrodiffusion 2_{out,1} , 2_{out,2} , 2_{out,3} décrit tout ou partie d'un anneau, de faible épaisseur dans le plan XY, de préférence inférieure à 1 mm, voire à 500 µm, voire 100 µm ou 50 µm et s'étendant autour de la zone d'illumination 2ᵢₙ. L'épaisseur est une épaisseur radiale, définie selon un rayon du collecteur passant par le centre C.

Comme mentionné dans EP3054281, plus la distance de rétrodiffusion est faible, plus le trajet, dans l'objet, des photons formant le rayonnement rétrodiffusé, s'étend faiblement en profondeur dans l'objet. Ainsi, plus la distance de rétrodiffusion est faible, plus le rayonnement rétrodiffusé est représentatif d'une partie superficielle de l'objet. A l'inverse, plus la distance de rétrodiffusion est importante, plus le rayonnement rétrodiffusé est représentatif d'une partie profonde de l'objet. Sur la figure 1A, on a segmenté l'objet en trois tranches adjacentes :
- une tranche superficielle, s'étendant jusqu'à une profondeur z₁ ;
- une tranche intermédiaire, s'étendant entre la profondeur z₁ et la profondeur z₂ ;
- une tranche profonde, s'étendant entre la profondeur z₂ et la profondeur z₃ ;

Le rayonnement rétrodiffusé 12₁, émanant de la surface de l'objet à la distance de rétrodiffusion d₁, ne traverse que la tranche superficielle : il est représentatif de la composition de l'objet uniquement dans la tranche superficielle. Le rayonnement rétrodiffusé 12₂, émanant de la surface de l'objet à la distance de rétrodiffusion d₂, traverse la tranche superficielle et la tranche intermédiaire : il est représentatif de la composition de l'objet uniquement dans ces deux tranches. On comprend que le rayonnement rétrodiffusé 12₃, émanant de la surface de l'objet à la distance de rétrodiffusion d₃, est représentatif de la composition de l'objet dans les trois tranches.

L'intensité du rayonnement rétrodiffusé à une distance de rétrodiffusion donnée est faible. Dans la publication Senlik O. et al « Concentric Multipixel Silicon Photodiode Array probes for Spatially resolved diffuse reflectance spectrometry », on décrit le recours à des photodiodes formant des secteurs annulaires, et destinées à être appliquées contre la surface d'un objet. Une telle solution suppose l'utilisation de photodiodes de surface élevée, ce qui augmente le bruit de détection. Ainsi, on augmente le signal utile, mais également le bruit de détection. Cela n'est pas optimal du point de vue du rapport signal sur bruit.

Les inventeurs proposent un dispositif permettant d'accroître la surface de collection du rayonnement rétrodiffusé, tout en limitant le bruit de détection du photodétecteur. Pour cela, un composant important du dispositif est un collecteur annulaire, décrivant un secteur annulaire, configuré pour collecter le rayonnement rétrodiffusé à une même distance de rétrodiffusion, et pour diriger le rayonnement rétrodiffusé vers un photodétecteur ponctuel. Par photodétecteur ponctuel, on entend de faible surface, par exemple inférieure à 10 µm par 10 µm, voire inférieure à 5 µm par 5 µm, idéalement de l'ordre de 1 µm par 1 µm.

Par secteur annulaire, on entend une région comprise entre deux cercles concentriques et deux rayons délimitant un angle donné. Il s'agit donc d'une "tranche" ou portion d'un anneau circulaire.

L'anneau ou le secteur annulaire s'étend autour d'un centre C. La largeur de l'anneau, dans le plan XY, est suffisamment faible pour que l'anneau soit considéré comme s'étendant à une même distance de la source de lumière. Ainsi, la largeur de l'anneau est de préférence inférieure à au moins 1/10^{ième}, voire 1/20^{ième} de la distance le séparant du centre C.

Dans l'exemple représenté, le dispositif comporte trois collecteurs 20₁, 20₂, 20₃, de largeur δ =25 µm, et espacés les uns des autres d'un écartement δ' de 100 µm ou quelques centaines de 100 µm. Les trois collecteurs 20₁, 20₂, 20₃ sont disposés respectivement à des distances d₁ = 250 µm, d₂ = 500 µm, d₃ = 750 µm du centre C. Lorsque le dispositif est mis en œuvre, le centre C est disposé dans la zone d'illumination 2ᵢₙ, au contact de la surface de l'objet 2.

Chaque distance de rétrodiffusion est généralement comprise entre 100 µm et quelques cm, de préférence entre 100 µm et 1 cm.

Dans la configuration représentée, chaque collecteur s'étend de part et d'autre d'un axe médian Δ, passant par le centre C et formant un axe de symétrie du collecteur. Chaque collecteur s'étend, par rapport à l'axe médian, et de part et d'autre de ce dernier, selon un angle d'extension θ pouvant atteindre ou dépasser 45°. L'angle d'extension θ peut atteindre π (anneau complet), mais les inventeurs considèrent qu'il est préférable que θ soit inférieur à 90°, voire à 60° ou 45°. En théorie, plus θ est élevé, plus la surface de collecte est importante, et plus l'intensité collectée du rayonnement rétrodiffusé est élevée. Cependant, la recherche d'un angle trop élevé peut impliquer des contraintes technologiques. Chaque collecteur est configuré pour focaliser le rayonnement rétrodiffusé vers un photodétecteur. Ainsi, les collecteurs 20₁, 20₂, 20₃ focalisent respectivement les rayonnements rétrodiffusés 12₁, 12₂ et 12₃ vers les photodétecteurs 22₁, 22₂ et 22₃. En aval de chaque collecteur 20₁, 20₂, 20₃, les rayonnements collectés 21₁, 21₂ et 21₃ convergent respectivement vers les photodétecteurs 22₁, 22₂ et 22₃. Chaque collecteur est ainsi optiquement couplé à un photodétecteur différent d'un autre collecteur. Le couplage entre chaque collecteur et chaque photodétecteur est bijectif, au sens ou un collecteur est optiquement couplé à un seul photodétecteur et réciproquement.

Le recours à des couples collecteur élémentaire / photodétecteur permet de détecter une éventuelle anomalie, par exemple lorsque les photodétecteurs respectivement couplés à des collecteurs, de même surface, et adressant une même distance de rétrodiffusion, détectent des intensités différentes. Par exemple, si l'un des photodétecteurs détecte une intensité plus faible que les autres, cela peut être la conséquence d'une singularité, par exemple poil ou zone ponctuelle absorbante. Le signal détecté, considéré comme anormal, peut alors être supprimé.

Les signaux des photodétecteurs adressant une même distance de rétrodiffusion peuvent être sommés, de façon à obtenir une seule intensité pour chaque distance de rétrodiffusion considérée.

Sur la configuration représentée sur la figure 1B, l'angle d'extension θ est le même pour les trois collecteurs. Selon une autre possibilité, les angles d'extension de collecteurs différents sont respectivement différents.

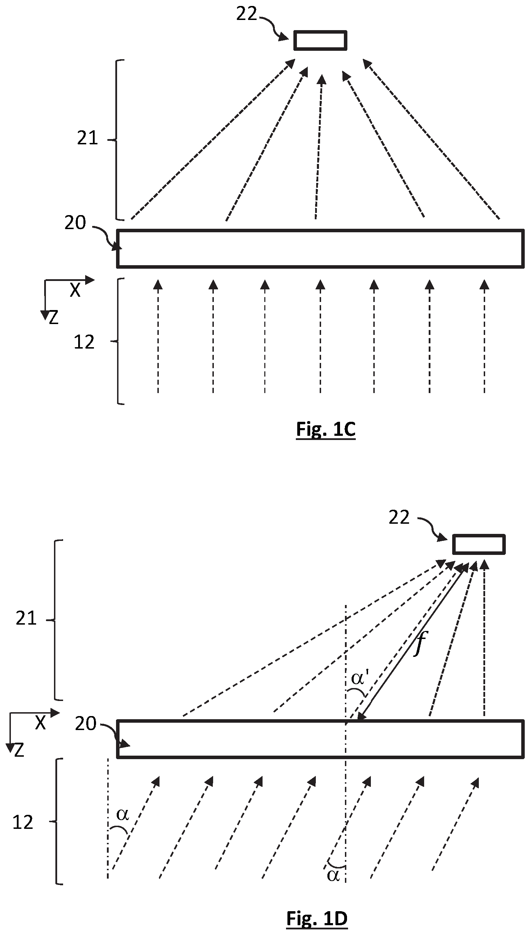

L'espace entre les collecteurs est opaque et absorbant dans la bande spectrale d'illumination Δλ, sauf au niveau du centre C, de façon à permettre au rayonnement d'illumination 11 de former la zone d'illumination 2ᵢₙ. Sur les figures 1C et 1D, on a représenté, dans le plan XZ, deux possibilités différentes de positionnement d'un photodétecteur 22 par rapport à un collecteur 20. La figure 1C représente une configuration dans laquelle un photodétecteur 22 est disposé de façon centrée à l'aplomb d'un collecteur 20. Le rayonnement collecté 21 converge vers le photodétecteur 22 en formant un cône symétrique. Une telle configuration est adaptée à la collection d'un rayonnement rétrodiffusé 12 émanant de l'objet selon une direction normale à la surface S. La figure 1D représente une configuration selon laquelle le photodétecteur 22 est disposé de façon décentrée par rapport à la configuration représentée sur la figure 1C. Ainsi, le photodétecteur 22 est décalé par rapport à la normale au collecteur 20, passant par le centre de ce dernier. Une telle configuration est adaptée à la collection d'un rayonnement rétrodiffusé 12 émanant de l'objet en étant incliné, selon un angle d'inclinaison α, par rapport à la direction normale à la surface S. Le rayonnement collecté 21 converge vers le photodétecteur 22 en formant un cône s'étendant autour d'un axe central incliné d'un angle α' par rapport à la normale à la surface de détection. Sur les figures 1C et 1D, la normale à la surface S est représentée en tirets mixtes.

En l'absence de déviation de l'axe autour duquel se propage le rayonnement rétrodiffusé, à travers la surface de détection, α et α' sont égaux.

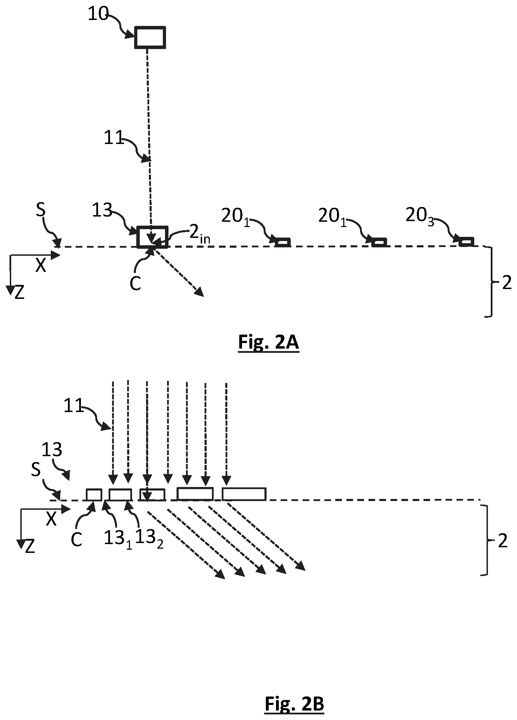

Les figures 2A et 2B illustrent une variante, selon laquelle le dispositif comporte un déflecteur 13, destiné à dévier le faisceau d'illumination 11 en direction des collecteurs. Le déflecteur 13 peut être formé par un prisme, ou par une microstructure optique agencée pour dévier le faisceau. Un exemple d'une telle microstructure est schématisée sur la figure 2B. Elle comporte des plots 13₂, d'épaisseur de l'ordre de la longueur d'onde, et de dimension latérale augmentant progressivement en s'éloignant du centre C, vers le ou les collecteurs. Plus précisément, la dimension latérale augmente le long d'un axe perpendiculaire (ici l'axe X) à l'axe de propagation du faisceau d'illumination 11, entre le centre C et le ou les collecteurs. Selon l'axe Y, le réseau est invariant. Les plots 13₂ sont espacés d'un pas spatial inférieur à la longueur d'onde d'illumination. Les plots sont formés par un matériau, de préférence non métallique, transparent dans la longueur d'onde d'illumination, et présentant un indice de réfraction supérieur à l'indice de réfraction du milieu ambiant 13₁. L'augmentation progressive de la dimension latérale des plots 13₂, d'indice de réfraction élevé, permet une déviation du faisceau d'illumination 11, idéalement de l'ordre de 45°.

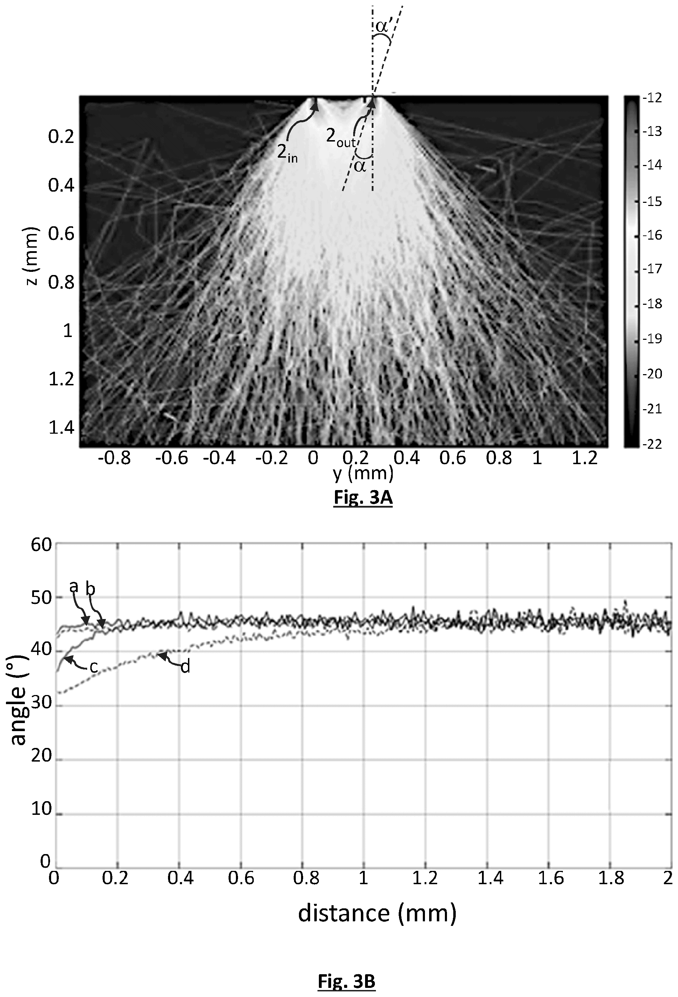

La figure 3A représente une modélisation du transport de photons dans un échantillon diffusant formé de lait, en prenant en compte une distance de rétrodiffusion de 250 µm. Il est rappelé que la distance de rétrodiffusion correspond à la distance entre le faisceau d'illumination et la zone de la surface de l'objet de laquelle émane le rayonnement rétrodiffusé. En fonction de la profondeur de pénétration dans l'objet, l'angle d'inclinaison α peut varier. L'angle d'inclinaison α peut être modélisé, ce qui permet de prévoir la position de chaque photodétecteur 22 relativement au collecteur 20 auquel il est couplé. Le photodétecteur est de préférence disposé dans l'axe optique du collecteur 20, incliné de l'axe α' par rapport à la normale à la surface de l'échantillon. L'angle α' correspond à l'inclinaison de l'axe reliant le centre du collecteur au centre du photodétecteur.

L'angle α dépend de la distance de rétrodiffusion. Sur la figure 3B, on a représenté l'angle α (axe des ordonnées - unité degré) en fonction de la distance de rétrodiffusion (axe des abscisses - unité mm), en utilisant différentes sources de lumière.
- courbe a : source de lumière lambertienne, se propageant selon un axe de propagation normal à la surface de l'échantillon.
- courbe b : source de lumière lambertienne, se propageant selon un axe de propagation incliné de 45° par rapport à la surface de l'échantillon.
- courbe c : source de lumière collimatée, se propageant selon un axe de propagation normal à la surface de l'échantillon.
- courbe d : source de lumière collimatée, se propageant selon un axe de propagation incliné de 45° par rapport à la surface de l'échantillon.

On constate quel que soit le type de source de lumière que plus la distance de rétrodiffusion augmente, plus l'angle α du rayonnement rétrodiffusé, par rapport à la normale à la surface de l'échantillon, se rapproche de 45°. En réalité, comme on peut le voir sur la figure 3A, l'inclinaison du rayonnement de rétrodiffusé est distribuée autour de l'angle α, ce dernier correspondant à l'angle d'inclinaison pour lequel le rayonnement rétrodiffusé est le plus intense.

Chaque photodétecteur est de préférence ponctuel, ou pouvant être considéré comme tel. Il peut par exemple s'agir d'une photodiode. La surface de détection du photodétecteur est de préférence de l'ordre ou inférieure à quelques µm² ou dizaines de µm². Alternativement, chaque photodétecteur peut être formé par une extrémité d'une fibre optique, pour guider la lumière jusqu'à un détecteur distant, par exemple un capteur d'image.

Selon une première possibilité, chaque collecteur prend la forme d'une plaque de zone de Fresnel, usuellement désignée par l'acronyme FZP (Fresnel Zone Plate). Une FZP est formée de portions concentriques alternant entre des portions transparentes ou opaques dans la bande spectrale d'illumination Δλ. Chaque portion est dimensionnée pour former des interférences constructives au niveau d'un point focal, permettant ainsi de concentrer l'énergie lumineuse incidente. On comprend que le point focal correspond au photodétecteur auquel le collecteur est optiquement couplé. Le principe de fonctionnement de FZP conformes avec l'invention a été décrit dans la publication Moreno V. et al, « High efficiency diffractive lenses : Deduction of kinoform profile », Am. J. Phys. 65 (6), June 1997.

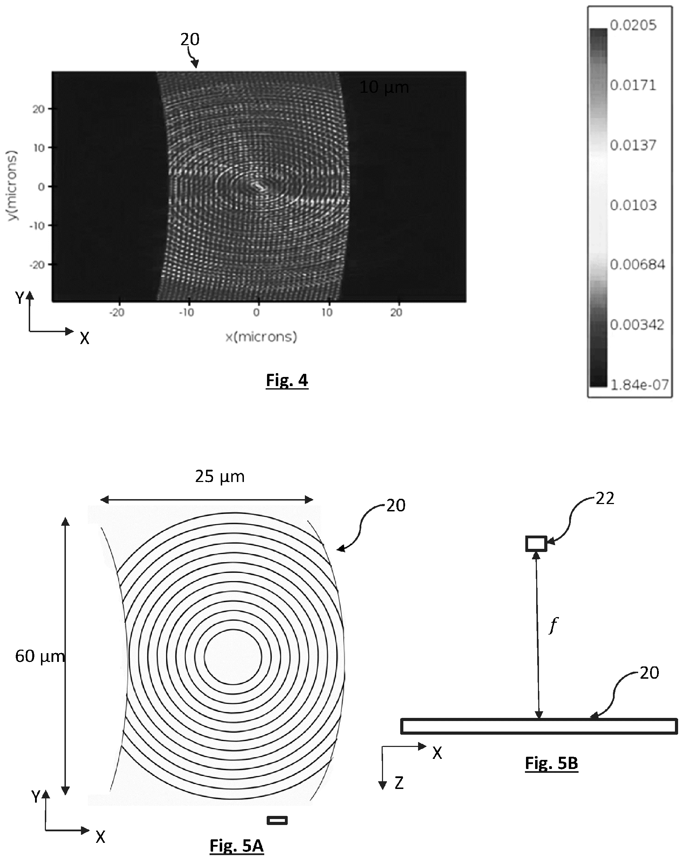

La figure 4 représente un exemple de collecteur 20, de type FZP, s'étendant selon une largeur de 25 µm et une hauteur de 60 µm. Un tel collecteur peut être obtenu par une succession d'anneaux concentriques auxquels est affecté un rang m, m étant un entier compris entre 1 et M, M correspondant à l'anneau de rayon maximal. Chaque anneau de rang m de part et d'autre d'un rayon *Rₘ* tel que *Rₘ*² = *mλf* , où *f* correspond à la focale, c'est-à-dire la distance entre le photodétecteur et le collecteur, selon l'axe optique. *λ* est la longueur d'onde du faisceau d'illumination.

Les matériaux formant les anneaux sont alternativement opaques et transparents dans la bande spectrale d'illumination Δλ. Cependant, l'efficacité de structures de type FZP est limitée, du fait de la présence de portions opaques. L'efficacité de collection peut être améliorée en utilisant des portions formées par une alternance de matériaux présentant un fort indice et un faible indice. Parmi les matériaux de faible indice, on peut choisir l'air ou la silice (indice de réfraction 1.5). Parmi les matériaux de fort indice, on peut choisir le silicium (indice de réfraction 3.5), ou le nitrure de silicium (1.7). Les portions de faible et fort indices induisent respectivement un déphasage de π et de 2π. En dehors du secteur annulaire 20, un dépôt métallique permet d'obtenir une opacité. L'échelle de niveau de gris est l'intensité lumineuse. La distribution de l'intensité lumineuse a été obtenue par modélisation avec une méthode de type FDTD (Finite-Différence Time Domain : méthode des différences finies dans le domaine temporel). L'épaisseur de chaque anneau de fort indice peut être de l'ordre de 1 µm.

La figure 5A représente une vue de dessus du collecteur, dans le plan XY, sur lequel les anneaux concentriques formant le FZP sont visibles à l'extérieur du secteur annulaire formant le collecteur. La réalisation du collecteur comporte la formation des anneaux concentriques, puis l'apposition d'un dépôt métallique en dehors du secteur annulaire formant le collecteur.

La figure 5B est une vue transversale, selon le plan XZ, de l'ensemble formé par le collecteur 20, représenté sur la figure 5A, et un photodétecteur 22, formant le point focal du collecteur 20. La dimension du photodétecteur est par exemple de 10 µm x 10 µm.

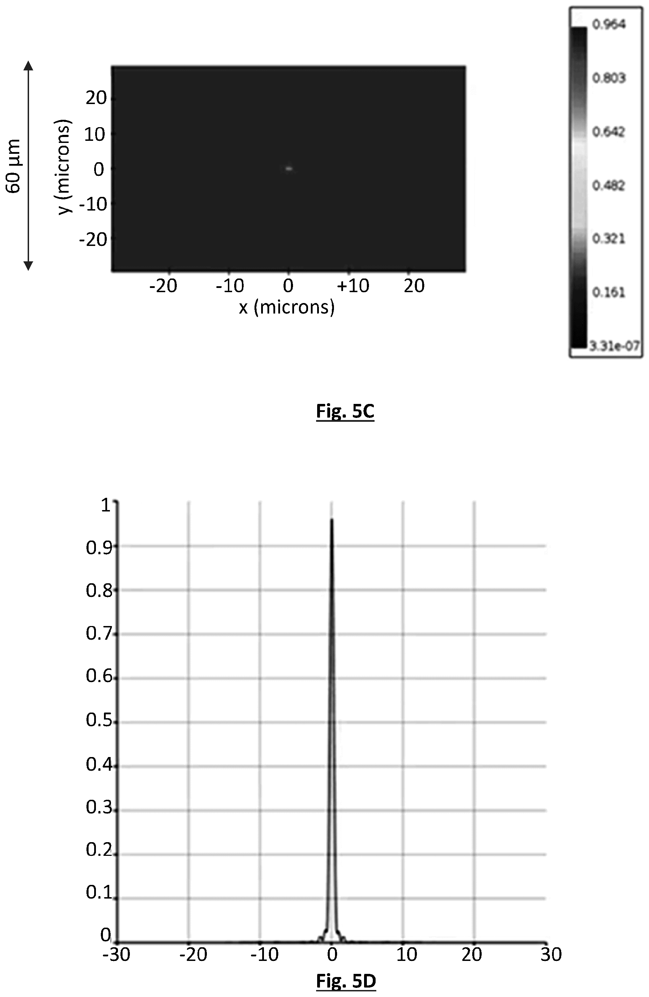

Sur la figure 5C, on a représenté l'intensité lumineuse dans un plan XY passant par le photodétecteur schématisé sur la figure 5B. L'échelle de niveau de gris correspond à l'intensité lumineuse. La figure 5C a été obtenue par modélisation, de la même façon que la figure 3A. On observe que la lumière collectée est concentrée sur une zone ponctuelle, au centre de l'image. La figure 5D est un profil d'intensité, le long d'une ligne passant par le point de focalisation de la figure 5C. Le profil permet d'apprécier la concentration de la lumière.

Selon une autre possibilité, le collecteur est formé de cristaux photoniques ou d'une métasurface. L'utilisation de structures périodiques qui modulent la propagation de la lumière, permettant le contrôle de la propagation de photons, est bien connue. De telles structures permettent notamment une focalisation de la lumière. Le recours à des cristaux photoniques bidimensionnels permet d'obtenir des « lentilles plates », qui focalisent la lumière vers un point focal précis, en exploitant les propriétés de diffraction et de réfraction des structures périodiques.

Quelle que soit la structure du collecteur, en présence de différents collecteurs, chaque collecteur focalise le rayonnement rétrodiffusé collecté vers un point focal qui lui est propre, et différent du point focal des autres collecteurs.

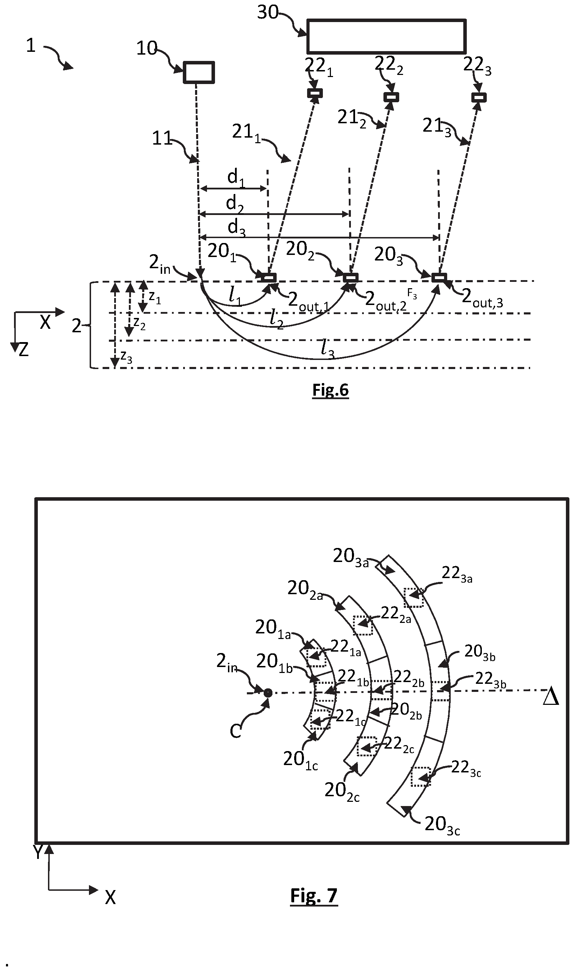

L'intensité d'un rayonnement rétrodiffusé à la distance *d*, à la longueur d'onde *λ* peut être explicitée selon : $I \left(λ, d, l\right) = {I}_{0} \left(λ\right) . R \left(λ, d\right) . {e}^{- {μ}_{t} l} {e}^{- {δμ}_{c} {c}_{c} l}$ (1) où :
- *I*₀(*λ*) est l'intensité du faisceau d'illumination atteignant la zone d'illumination 2ᵢₙ.
- *R*(*λ, d*) est la réflectance de l'objet, sans absorption, à la longueur d'onde *λ*, liée à la présence de diffuseurs dans l'objet, à la distance de rétrodiffusion *d* ;
- *e*^{*-µₜₗ*(*d*)} est l'absorption du rayonnement par l'objet, de coefficient d'absorption *µₜ*, le long de la trajectoire *l*(*d*) du rayonnement dans l'objet. La trajectoire *l*(*d*) correspond au chemin moyen parcouru par les photons émergeant de l'objet à la distance de rétrodiffusion *d*, en tenant compte de la courbure matérialisée par les flèches *l*₁, *l*₂, *l*₃ sur la figure 6.
- *e*^{*-δµ_{cc}cₗ*(*d*)} est la variation du signal engendré par la présence d'une molécule de concentration *c_{c}* et d'absorption linéique *δµ_{c}* le long de la trajectoire *l*(*d*) suivie par le rayonnement.

Dans l'application décrite, on cherche à déterminer une modification de l'absorption du milieu en présence d'une molécule d'intérêt induisant une variation *δµ_{c}* de l'absorption.

La trajectoire *l*(*d*) peut être considérée comme proportionnelle à la distance de rétrodiffusion *d* : *l*(*d*) = *σd* (2) où *σ* est un coefficient réel positif. Ainsi : $I \left(λ, d\right) = {I}_{0} \left(λ\right) . R \left(λ, d\right) . {e}^{- {μ}_{t} σd} {e}^{- {δμ}_{c} {c}_{c} σd}$ (3)

L'intensité détectée par chaque photodétecteur est notée *i*(*λ*, *d*). Elle dépend d'un facteur de concentration *K*(*d*) de chaque concentrateur qui correspond à la capacité d'un concentrateur à concentrer la lumière collectée sur le photodétecteur auquel il est couplé.

Ainsi : $i \left(λ, d\right) = {I}_{0} \left(λ\right) . R \left(λ, d\right) . K \left(d\right) . {e}^{- {μ}_{t} σd} {e}^{- {δμ}_{c} {c}_{c} σd}$ (5)

Pour chaque distance de rétrodiffusion *d*, on peut dimensionner le collecteur de façon que $K \left(d\right) = \frac{{K}_{0}}{R \left(λ, d\right) {e}^{- {μ}_{t} σd}}$ (6)

Les grandeurs *R*(*λ*, *d*) et *e^{-µₜσd}* peuvent être estimées par modélisation, ou par des mesures effectuées sur des fantômes mimant l'objet, en l'absence de chromophore.

*K*₀ est défini pour chaque collecteur, en fonction de l'extension maximale du premier anneau (*m* = 1), dont le rayon dépend de la focale et de la longueur d'onde. *K*₀ peut être estimé par modélisation et/ou par essais expérimentaux.

En prenant en compte (6), on peut disposer de plusieurs collecteurs, à différentes distances de rétrodiffusions *d*, et dont la dimension, notamment l'extension angulaire θ, permet de satisfaire à l'équation (6).

En combinant (5) et (6), l'intensité lumineuse détectée par chaque photodétecteur devient ainsi : $i \left(λ, d\right) = {I}_{0} \left(λ\right) . {K}_{0} {e}^{- {δμ}_{c} {c}_{c} σd}$ (7)

L'avantage de disposer de deux distances de rétrodiffusion est de pouvoir effectuer, au même instant, un ratio : $\frac{i \left(λ, {d}_{1}\right)}{i \left(λ, {d}_{2}\right)} = {e}^{- {δμ}_{c} {c}_{c} σ \left({d}_{1}-{d}_{2}\right)}$ (8)

Cela permet de s'affranchir de la connaissance du faisceau d'illumination, et des éventuelles variations de ce dernier.

La figure 7 illustre une variante selon laquelle chaque collecteur est segmenté en différents collecteurs élémentaires. Chaque collecteur élémentaire formant un collecteur est situé à une même distance du centre C. Sur la figure 7, on a représenté :
- Le premier collecteur 20₁, formé par trois premiers collecteurs élémentaires adjacents 20₁ₐ, 20_{1b}, 20_{1c} ;
- Le deuxième collecteur 20₂, formé par trois deuxièmes collecteurs élémentaires adjacents 20₂ₐ, 20_{2b}, 20_{2c} ;
- Le troisième collecteur 20₃, formé par trois deuxièmes collecteurs élémentaires adjacents 20₃ₐ, 20_{3b}, 20_{3c} ;

Chaque collecteur élémentaire est optiquement couplé à un photodétecteur élémentaire, selon un lien bijectif. Ainsi :
- Le premier photodétecteur 22₁ est formé par des premiers photodétecteurs élémentaires 22₁, 22_{1b} et 22_{1c} respectivement optiquement couplés aux premiers collecteurs élémentaires 20₁ₐ, 20_{1b}, 20_{1c}.
- Le deuxième photodétecteur 22₂ est formé par des deuxièmes photodétecteurs élémentaires 22₂ₐ, 22_{2b} et 22_{2c} respectivement optiquement couplés aux deuxièmes collecteurs élémentaires 20₂ₐ, 20_{2b}, 20_{2c}.
- Le troisième photodétecteur 22₃ est formé par des troisièmes photodétecteurs élémentaires 22₃ₐ, 22_{3b} et 22_{3c} respectivement optiquement couplés aux troisièmes collecteurs élémentaires 20₃ₐ, 20_{3b}, 20_{3c}.

Les photodétecteurs élémentaires d'un même photodétecteur sont reliés entre eux, de façon que l'intensité détectée par chaque photodétecteur élémentaire soit sommée. Cela permet de cumuler les intensités respectivement détectés par les photodétecteurs élémentaires adressant une même distance de rétrodiffusion.

La subdivision d'un photodétecteur en plusieurs photodétecteurs élémentaires permet de s'affranchir des contraintes technologiques liées à la formation d'un collecteur dont l'extension angulaire est trop élevée. On peut ainsi augmenter la surface de collecte, et donc l'intensité du rayonnement rétrodiffusé détectée, en mettant en œuvre des collecteurs élémentaires de conception simple. L'extension angulaire de chaque collecteur élémentaire peut être inférieure à 45°, voire inférieure à 30°, voire inférieure à 15° ou à 10°.

Les photodétecteurs sont reliés à une unité de traitement 30, configurée pour traiter les signaux de détection résultant de chaque photodétecteur. L'unité de traitement peut comporter des modules analogiques ou numériques pour le traitement des signaux de détection, ainsi qu'un microprocesseur, pour appliquer un algorithme d'analyse des signaux de détection traités, par exemple pour déterminer une concentration d'un chromophore, ou un autre paramètre physiologique traduit par les signaux de détection.

Selon une possibilité, le module analogique comporte des amplificateurs logarithmiques, qui convertissent un signal d'entrée en une tension de sortie proportionnelle à l'amplitude du signal d'entrée. Chaque amplificateur logarithmique est configuré pour être alimenté par le signal de détection d'un photodétecteur.

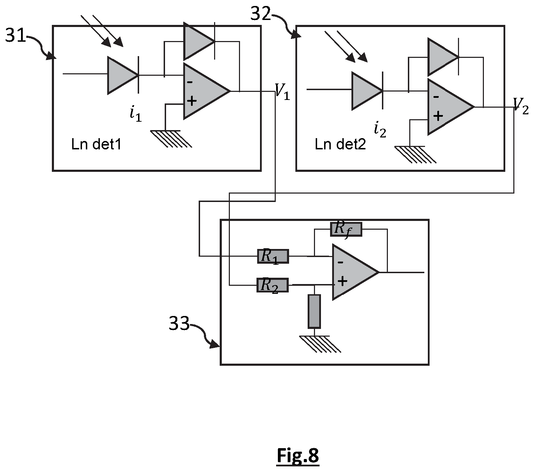

Sur la figure 8, on a représenté :
- un premier amplificateur logarithmique 31, alimenté par le signal de détection *i*₁ correspondant à la première distance de rétrodiffusion *d*₁. La tension de sortie est :${V}_{1} ∞ ln \left({i}_{1}\right)$ (9);
- un deuxième amplificateur logarithmique 32, alimenté par le signal de détection *i*₂ correspondant à la deuxième distance de rétrodiffusion *d*₂. La tension de sortie est :${V}_{2} ∞ ln \left({i}_{2}\right)$ (9');
- un soustracteur 33, alimenté par les premier et deuxième amplificateurs logarithmiques dont la tension de sortie est : ${V}_{s} = \frac{{R}_{f}}{{R}_{1}} \left({V}_{2}-{V}_{1}\right)$ (10).

En combinant (9), (9') et (10), on obtient : ${V}_{s} = \frac{{R}_{f}}{{R}_{1}} {δμ}_{c} {c}_{c} σ \left({d}_{2}-{d}_{1}\right)$ (11)

Dans la figure 8, on suppose que *R*₂ = *R*₁.

Ainsi, la mesure de *Vₛ* permet d'obtenir la concentration *c_{c}* de chromophore recherchée. Le montage représenté sur la figure 8 permet d'isoler le signal utile, en l'occurrence *Vₛ*, avant de procéder à son traitement, par exemple son amplification.

Le recours à des concentrateurs permet d'augmenter la surface de collecte, tout en utilisant un photodétecteur de taille réduite, ce qui permet d'augmenter le signal détecté tout en conservant un niveau de bruit faible. En effet, le courant d'obscurité d'un photodétecteur peut être considéré comme proportionnel à sa surface. Le rapport signal à bruit est augmenté par rapport aux configurations de l'art antérieur. Cela permet d'améliorer la sensibilité du dispositif lorsqu'il s'agit de d'estimer une concentration d'un chromophore. Cela permet également de déterminer les propriétés d'absorption ou de diffusion avec plus de précision.

En outre, lorsque la source de lumière est cohérente, des interférences constructives ou destructives peuvent entraîner l'apparition de figures de diffraction, ou speckle, affectant l'uniformité spatiale du rayonnement rétrodiffusé à une distance de rétrodiffusion donnée. Le recours à un collecteur permet d'augmenter la surface de collection, et de lisser une éventuelle non uniformité spatiale du rayonnement rétrodiffusé.

Chaque collecteur est de préférence délimité par au moins une surface plane. Il peut être disposé au contact de l'échantillon, ou sur une couche d'un matériau intercalaire permettant une adaptation de l'indice optique.

L'invention peut être mise en œuvre sur un tissu biologique, pour les applications décrites dans l'art antérieur. Elle peut également être mise en œuvre pour analyser des échantillons, par exemple dans l'industrie cosmétique ou agroalimentaire.

## Revendications

1. Dispositif de mesure d'un rayonnement rétrodiffusé (12, 12₁, 12₂, 12₃, 12₄) par un objet (2) comportant :
- au moins une source de lumière (10) configurée pour émettre un faisceau lumineux (11), dans une bande spectrale d'illumination (Δλ), vers une surface (S) délimitant l'objet, de façon à former, sur ladite surface, une zone d'illumination (2ᵢₙ) ;
- un photodétecteur (22, 22₁, 22₂, 22₃, 22₄), pour détecter une intensité du rayonnement rétrodiffusé par l'objet, lorsque ce dernier est illuminé par la source de lumière, le photodétecteur s'étendant selon une surface de détection ;
le dispositif étant **caractérisé en ce qu'**il comporte :
- un collecteur de rayonnement (20, 20₁, 20₂, 20₃, 20₄), s'étendant selon une surface de collection, la surface de collection décrivant au moins un secteur annulaire s'étendant autour d'un centre (C), la surface de collection étant plus grande que la surface de détection ;
- et **en ce que** lorsque la surface de collection est disposée face ou contre la surface de l'objet, le collecteur de rayonnement est configuré pour focaliser un rayonnement, incident à la surface de collection, vers le photodétecteur, le collecteur étant optiquement couplé au photodétecteur ;
- de façon que lorsque le centre (C), autour duquel s'étend le collecteur, est disposé dans la zone d'illumination (2ᵢₙ), le collecteur est agencé pour focaliser le rayonnement rétrodiffusé (12, 12₁, 12₂, 12₃, 12₄), incident à la surface de collection, vers le photodétecteur.

2. Dispositif selon la revendication 1, dans lequel le ou chaque collecteur est formé d'une plaque de zone de Fresnel, le collecteur étant formé de portions concentriques alternant entre des portions transparentes et opaques, configurées pour focaliser le rayonnement incident au collecteur vers le photodétecteur auquel ledit collecteur est optiquement couplé.

3. Dispositif selon la revendication 1, dans lequel le ou chaque collecteur est formé de portions concentriques alternant entre des portions respectivement d'un premier indice et d'un deuxième indice, supérieur au premier indice, et configurée pour focaliser le rayonnement incident au collecteur vers le photodétecteur auquel ledit collecteur est optiquement couplé.

4. Dispositif selon la revendication 1, dans lequel le ou chaque collecteur est formé d'une structure optique, de type métasurface ou cristaux photoniques bidimensionnels, configurée pour focaliser le rayonnement incident au collecteur vers le photodétecteur auquel ledit collecteur est optiquement couplé.

5. Dispositif selon la revendication 1, dans lequel le collecteur s'étend à une distance, dite distance de rétrodiffusion, du centre C, de façon à focaliser le rayonnement rétrodiffusé par l'objet, à ladite distance de rétrodiffusion, vers le photodétecteur.

6. Dispositif selon l'une quelconque des revendications précédentes, comportant :
- plusieurs photodétecteurs (22₁, 22₂, 22₃, 22₄);
- plusieurs collecteurs concentriques (20₁, 20₂, 20₃, 20₄), s'étendant autour du centre, chaque collecteur étant disposée à une distance du centre (C) différente des autres collecteurs, chaque collecteur étant optiquement couplé à un photodétecteur différent.

7. Dispositif selon l'une quelconque des revendications précédentes, dans lequel la surface de collection est inférieure à 10 mm² ou à 5 mm² ou à 1 mm².

8. Dispositif selon l'une quelconque des revendications précédentes, dans lequel chaque photodétecteur est disposé face au collecteur auquel il est couplé.

9. Dispositif selon l'une quelconque des revendications précédentes, dans lequel la largeur du ou de chaque secteur angulaire, radialement, est inférieure à 500 µm ou 250 µm ou 100 µm.

10. Dispositif selon l'une quelconque des revendications précédentes, dans lequel :
- au moins un collecteur est subdivisé en plusieurs collecteurs élémentaires, s'étendant à une même distance du centre ;
- chaque collecteur élémentaire est optiquement couplé à un photodétecteur élémentaire, de façon que chaque photodétecteur élémentaire forme le photodétecteur.

11. Dispositif selon la revendication 10, dans lequel chaque collecteur élémentaire s'étend selon une extension angulaire, autour du centre, inférieure à 90° ou inférieure à 45° ou inférieure à 30° ou inférieure à 15°.

12. Dispositif selon l'une quelconque des revendications précédentes, comportant un déflecteur (13) , configuré pour dévier, le faisceau lumineux, atteignant l'objet, vers au moins un collecteur.

13. Dispositif selon l'une quelconque des revendications précédentes, dans lequel la bande spectrale d'illumination s'étend dans le domaine visible ou dans l'infra-rouge.

14. Procédé de détection d'un rayonnement rétrodiffusé par un objet, à l'aide d'un dispositif selon l'une quelconque des revendications précédentes, le procédé comportant les étapes suivantes :
- a) placement du dispositif sur l'objet, de façon que le centre soit situé dans la zone d'illumination et que le ou chaque collecteur soit disposé contre ou face à la surface de l'objet ;
- b) illumination de l'objet à l'aide de la source de lumière, de façon à former, sur la surface de l'objet, une zone d'illumination,
- c) détection, par le ou chaque photodétecteur, du rayonnement rétrodiffusé par l'objet, et focalisé par le collecteur optiquement couplé audit photodétecteur.

15. Procédé selon la revendication 14, dans lequel le procédé comporte :
- une détermination d'une propriété optique de diffusion et/ou d'absorption en fonction d'une intensité du rayonnement rétrodiffusé, à différentes distances de rétrodiffusion.
- ou une détermination d'une concentration d'une espèce absorbant la lumière dans la bande spectrale d'illumination.
